# DEMANDE DE BREVET EUROPEEN

(11) **EP 0 911 404 A2**
(43) Date de publication de la demande: **28.04.1999**
(21) Numéro de dépôt: 98118052.4
(22) Date de dépôt: 23.09.1998
(51) Int. Cl.: C12N 15/53, C12N 9/02, C12N 15/67, C12N 15/81

(54) **Recodage de sequences d ADN permettant leur expression dans les levures et levures transformees obtenues**

(30) Priorité: 24.09.1997 FR 9712094
(71) Demandeur: Rhone Poulenc Agro, 69009 Lyon (FR)
(72) Inventeur: Batard, Yannick, 67000 Strasbourg (FR); Durst, Francis, 67170 Bernolsheim (FR); Schalk, Michel, 67230 Hutteheim (FR); Werck-Reichhart Danièle, 67370 Dingsheim (FR)
(74) Mandataire: Tetaz, Franck

(57) **Abrégé**

La présente invention concerne une séquence d'ADN codant pour une protéine d'intérêt contenant des régions à forte teneur en codons mal adaptés aux levures, caractérisée en ce qu'un nombre suffisant de codons mal adaptés aux levures est remplacé par des codons correspondants adaptés aux levures dans lesdites régions à forte teneur en codons mal adaptés aux levures.

La présente invention concerne plus particulièrement les séquences d'ADN d'origine de plantes dicotylédones ou monocotyledones, en particulier de plantes de la famille des graminées, en particulier choisies parmi le blé, l'orge, l'avoine, le riz, le maïs, le sorgho ou la canne à sucre.

La présente invention concerne également les levures transformées comprenant une séquence d'ADN selon l'invention,.

## Description

La présente invention concerne le recodage des séquences d'ADN codant pour des protéines contenant des régions à forte teneur en codons mal traduits par les levures, en particulier codant pour des protéines d'origine végétale, comme les cytochromes P450 d'origine végétale, et leur expression dans les levures.

Il est connu que certaines séquences, codant pour des protéines d'intérêt, notamment d'origine végétale, sont difficilement traduites dans les levures. C'est le cas en particulier pour les protéines ayant des régions à forte teneur en codons mal adaptés aux levures, en particulier des codons leucine, comme certains cytochromes P450 d'origine végétale. Certains systèmes développés pour améliorer l'expression de cytochromes P450 d'origine animale ou végétale dans les levures, comme ceux décrits par Pompon & coll.(*Methods Enzymol*, 272, 1996, 51-64; WO 97/10344), se sont révélés inadaptés pour de nombreux cytochromes P450 comprenant des régions à forte teneur en codons mal adaptés aux levures.

Les cytochromes P450 constituent une super famille d'enzymes membranaires, de type monooxygénase capables d'oxyder une grande famille de substrats généralement hydrophobes. Les réactions se caractérisent le plus souvent par l'oxydation des liaisons C-H ou C=C, des hétéroatomes, plus rarement par réduction des groupes nitro ou par déshalogénation. Ces enzymes interviennent plus particulièrement dans le métabolisme des xénobiotiques et des médicaments, et dans la biosynthèse de métabolites secondaires chez les plantes, dont certains à propriétés organoleptiques ou pharmacodynamiques.

Les cytochromes P450 sont en conséquence utilisés notamment dans:
- le diagnostic *in vitro* de la formation de métabolites toxiques ou mutagènes (molécules d'origine naturelle, polluants, médicaments, pesticides, etc.) permettant en particulier le développement de nouvelle molécules actives (pharmacie, agrochimie),
- l'identification et la destruction de molécules toxiques ou polluantes pour l'environnement,
- la synthèse enzymatique de nouvelles molécules.

La recherche d'une expression hétérologue des cytochromes P450 par des cellules hôtes, et plus particulièrement des levures est donc importante pour obtenir une production contrôlée de cette enzyme en grande quantité, soit pour l'isoler et l'utiliser dans les opérations énoncées ci-dessus, soit pour employer directement les cellules transformées pour lesdites opérations sans préalablement isoler l'enzyme.

La présente invention apporte une solution au problème énoncé ci-dessus, permettant l'expression dans des levures de protéines contenant des régions à forte teneur en codons mal adaptés aux levures, et en particulier des cytochromes P450 d'origine végétale.

La présente invention concerne donc une séquence d'ADN, en particulier d'ADNc, codant pour une protéine d'intérêt contenant des régions à forte teneur en en codons mal adaptés aux levures, caractérisée en ce qu'un nombre suffisant de codons mal adaptés aux levures est remplacé par des codons correspondants adaptés aux levures dans lesdites régions à forte teneur en codons mal adaptés aux levures.

Au sens de la présente invention, on entend par " codons mal adaptés aux levures" les codons dont la féquence d'utilisation par les levures est inférieure ou égale à environ 13 pour 1000, de préférence inférieure ou égale à environ 12 pour 1000, plus préférentiellement inférieure ou égale à environ 10 pour 1000. La fréquence d'utilisation des codons par les levures, et plus particulièrement par *S. cerevisiae* est notamment décrite dans "Codon usage data base from Yasukazu Nakamura" (http://www.dna.affrc.go.jp/∼nakamura/codon.html). C'est le cas en particulier des codons CTC, CTG et CTT codant pour la leucine, des codons CGG, CGC, CGA, CGT et AGG codant pour l'arginine, des codons GCG et GCC codant pour l'alanine, des codons GGG, GGC et GGA codant pour la glycine et des codons CCG et CCC codant pour la proline. Les codons mal adaptés aux levures selon l'invention sont plus particulièrement les codons CTC et CTG codant pour la leucine, CGG, CGC, CGA, CGT et AGG codant pour l'arginine, les codon GCG et GCC pour l'alanine, GGG et GGC codant pour la glycine et les codons CCG et CCC codant pour la proline.

Au sens de la présente invention, on entend par "codons correspondants adaptés aux levures" les codons correspondants aux codons mal adaptés aux levures, codant pour les même acides aminés et dont la féquence d'utilisation par les levures est supérieure à 15 pour 1000, de préférence supérieure ou égale à 18 pour 1000, plus préférentiellement supérieure ou égale à 20 pour 1000. C'est le cas en particulier des codons TTG et TTA, préférentiellement TTG, codant pour la leucine, du codon AGA codant pour l'arginine, des codons GCT et GCA, préférentiellement GCT, codant pour l'alanine, du codon GGT codant pour la glycine et du codon CCA codant pour la proline.

Au sens de la présente invention, on entend par " région à forte teneur en en codons mal adaptés aux levures" toute région de la séquence d'ADN comprenant au moins 2 codons mal adaptés parmi 10 codons consécutifs, les deux codons pouvant être adjacents ou séparées par jusqu'à 8 autres codons. Selon un mode de réalisation préférentiel de l'invention, les régions à forte teneur en codons mal adaptés comprennent 2, 3, 4, 5 ou 6 codons mal adaptés pour 10 codons consécutifs, ou comprennent au moins 2 ou 3 codons mal adaptés adjacents.

Au sens de la présente invention, on entend par "nombre suffisant de codons", le nombre de codons à remplacer nécessaires et suffisants pour observer une amélioration substantielle de leur expression dans les levures. De manière avantageuse, au moins 50 % des codons mal adaptés aux levures de la région à forte teneur considérée sont remplacés par des codons adaptés. De manière préférentielle, au moins 75 % des codons mal adaptés de la dite région sont remplacés, plus préférentiellement 100 % des codons mal adaptés sont remplacés.

Au sens de la présente invention, on entend par "amélioration substantielle" soit une expression détectable lorsque aucune expression de la séquence de référence n'est observée, soit une augmentation de l'expression par rapport au niveau d'expression de la séquence de référence.

Au sens de la présente invention, on désigne par "séquence de référence" toute séquence codant pour une protéine d'intérêt, qui est modifiée selon l'invention pour favoriser son expression dans les levures.

La présente invention est particulièrement adaptée aux séquences d'ADN, en particuler d'ADNc, codant pour des protéines d'intérêt comprenant des régions à forte teneur en leucine pour lesquelles un nombre suffisant de codons CTC codant pour la leucine dans ladite région à forte teneur en leucine, est remplacé par des codons TTG et/ou TTA, ou pour lequelles un nombre suffisant de codons CTC et CTG codant pour la leucine dans ladite région à forte teneur en leucine, sont remplacés par des codons TTG et/ou TTA, de préférence par un codon TTG.

Au sens de la présente invention, on entend par " région à forte teneur en leucine" une région comprenant au moins 2 leucines parmi 10 acides aminés consécutifs dans la protéine d'intérêt, les deux leucines pouvant être adjacentes ou séparées par jusqu'à 8 autres acides aminés. Selon un mode de réalisation préférentiel de l'invention, les régions à forte teneur en leucine comprennent 2, 3, 4, 5 ou 6 leucines pour 10 acides aminés consécutifs, ou comprennent au moins 2 ou 3 leucines adjacentes.

Selon un mode de réalisation préférentiel de l'invention, au moins 50 % des codons CTC ou CTC et CTG de la région à forte teneur en leucine sont remplacés par des codons TTG ou TTA, de préférence au moins 75 % des codons CTC ou CTC et CTG de ladite région sont remplacés, plus préférentiellement 100 % des codons CTC ou CTC et CTG sont remplacés.

De manière avantageuse, la présente invention est particulièrement appropriée pour les séquences d'ADN dont la teneur générale en codons mal adaptés est d'au moins 20%, plus préférentiellement d'au moins 30%, par rapport au nombre total de codons dans la séquence de référence.

De manière avantageuse, lorsque la séquence de référence comprend au moins une région en 5' à forte teneur en codons mal adaptés, le recodage de cette seule région en 5' permet d'obtenir une amélioration substantielle de l'expression de la protéine d'intérêt dans les levures. La longueur de la région en 5' à recoder selon l'invention sera variable fonction de la longueur de la région à forte teneur en codons mal adaptés. Cette longueur sera avantageusement d'au moins quatre codons, en particulier lorsque cette région comprend au moins deux mauvais codons adjacents, jusqu'à environ 40 codons, ou plus.

Il n'est cependant pas nécessaire selon l'invention de recoder toute la séquence de référence, mais uniquement les régions à forte teneur en mauvais codons, en particulier la région en 5' seule, pour obtenir une amélioration substantielle de l'expression de la protéine d'intérêt dans les levures.

De manière avantageuse, la séquence d'ADN codant pour une protéine d'intérêt est une séquence d'ADN isolée d'origine naturelle, en particulier d'origine végétale. L'invention est particulièrement avantageuse pour les séquences d'origine de plantes mono ou dicotylédones, de préférence monocotylédones, en particulier de la famille des graminées comme par exemple le blé, l'orge, l'avoine, le riz, le maïs, le sorgho, la canne à sucre, etc.

Selon un mode préférentiel de réalisation de l'invention, la séquence d'ADN code pour une enzyme, en particulier un cytochrome P450, de préférence d'origine végétale. Ces cytochromes P450 présentant une forte teneur en codons mal adaptés, en particulier codant pour la leucine dans leur région N terminale, c'est dans la région codante de l'extrémité 5' que les codons mal adaptés sont remplacés.

La présente invention concerne également un gène chimère comprenant une séquence d'ADN modifiée ci-dessus et des éléments de régulation en position 5' et 3' hétérologues pouvant fonctionner dans une levure, c'est à dire capables de commander l'expression de la protéine d'intérêt dans la levure. De tels éléments de régulation sont bien connus de l'homme du métier, décrits notamment par Rozman & coll. (Genomics, 38, 1996, 371-381) et par Nacken & coll. (Gene, 175, 1996, 253-260, *Probing the limits of expression levels by varying promoters strength and plasmid copy number in Saccharomyces cerevisiae*).

La présente invention concerne également un vecteur pour la transformation des levures comprenant au moins un gène chimère ci-dessus. Elle concerne aussi un procédé de transformation des levures avec ledit vecteur, et les levures transformées obtenues. Elle concerne enfin un procédé de production d'une protéine d'intérêt hétérologue dans une levure transformée, la séquence codant pour ladite protéine d'intérêt étant telle que définie ci-dessus.

Le procédé de production d'une protéine d'intérêt hétérologue dans une levure transformée, comprenant les étapes de:
a) transformation d'une levure avec un vecteur capable de se repliquer dans les levures, comprenant une séquence d'ADN modifiée définie ci-dessus et des éléments de régulation en position 5' et 3' hétérologues pouvant fonctionner dans une levure,
b) culture de la levure transformée, et
c) extraction de la protéine d'intérêt de la culture de levure.

Dans le cas où la protéine d'intérêt est une enzyme appropriée pour la transformation d'un substrat, comme un cytochrome P450, l'enzyme extraite de la culture de levure est ensuite utilisée comme catalyseur de la transformation dudit substrat.

La catalyse peut toutefois être réalisée sans nécessiter l'extraction de la levure en cultivant la levure transformée en présence du dit substrat.

La présente invention concerne donc également un procédé de transformation d'un substrat par catalyse enzymatique au moyen d'une enzyme exprimée dans une levure, comprenant les étapes de
a) culture de la levure transformée selon l'invention en présence du substrat à transformer, puis
b) récupération du substrat transformé de la culture de levure.

Dans le cas de la levure transformée pour l'expression d'un cytochrome P450, la réaction catalysée par l'enzyme est une réaction d'oxydation, plus particulièrement des liaisons C-H ou C=C.

Les techniques de transformation et de culture des levures sont connues de l'homme du métier, notamment décrites dans *Methods in Enzymology* (vol. 194, 1991).

Les levures utiles selon l'invention sont notamment choisies parmi les genres *Saccharomyces, Kluyveromyces, Hansenula, Pichia et Yarrowia*. De manière avantageuse, la levure est du genre *Saccaromyces*, en particulier *S. cerevisiae*.

D'autres caractéristiques de l'invention apparaitront à la lumière des exemples ci-après.

### Exemple 1: Création d'une banque de gène d'ADNc de blé et identification de la séquence CYP73A17

La séquence de cytochrome P450 de blé CYP73A17 a été obtenue par criblage d'une banque d'ADNc de jeunes plantules de blé (pousses et racines sans les caryopses) construite dans le vecteur λ-ZapII (Stratagène) selon les instructions du fournisseur.

### 1. Création de la banque d'ADNc

Des graines de *Triticum aestivum* (L. cv. Darius) enrobées de cloquintacet-mexyl (0,1 % par poids sec de graines) sont cultivées dans des boites de plastique sur deux couches de gaze humide jusqu'à l'obtention de pousses d'une taille de 3 à 5 mm. L'eau dans les boites est ensuite remplacée par une solution de phénobarbital de sodium 4 mM et le blé est cultivé jusqu'à une taille des pousses d'environ 1 cm.

La banque d'ADNc est construite à partir de 5 µg d'ARN poly(A)⁺ (Lesot, A., Benveniste, I., Hasenfratz, M.P., Durst, F. (1990) Induction of NADPH cytochrome P450(c) reductase in wounded tissues from *Helianthus tuberosus* tubers. Plant Cell Physiol., 31, 1177-1182) isolés à partir des pousses et des racines traitées, dans le vecteur λ-ZapII (Stratagène) selon le protocole et les instructions du fournisseur.

### 2. Criblage de la banque d'ADNc

5x10⁵ plages de lyse de la banque λ-ZapII obtenue précédemment sont criblées avec une sonde correspondant à la séquence codante complète de CYP73A1 d'*Helianthus tuberosus* marquée par amorçage au hasard au [α-³²P]dCTP. La préhybridation et l'hybridation des filtres est effectuée à faible stringence à 55 °C selon les protocoles standards. Les membranes sont lavées deux fois pendant 10 minutes avec 2 x SSC, 0,1 % SDS, et une fois pendant 10 minutes avec 0,2 x SSC, 0,1 % SDS à température ambiante, puis deux fois pendant 30 minutes avec 0,2 x SSC, 0,1 % SDS à 45 °C. Les inserts des plages de lyse positives sont analysés par RPC (réaction de polymérisation en chaine) et hybridation afin de déterminer leur taille. Les clones avec des inserts s'hybridant à CYP73A1 dans les conditions décrites ci-dessus et d'une taille supérieure à 1,5 kbp sont recriblés, avant l'excision du plasmide pBluescript selon le protocole du fournisseur (Stratagène) et séquençage par la technique prism Ready Reaction Dye Deoxy Terminator Cycle développée par Applied Biosystems Inc. Un clone de longueur totale est alors identifié par alignement avec CYP73A1.

Le cytochrome P450 de blé CYP73A17 codé par la séquence isolée (identificateur de séquence N° 1) présente 76,2 % d'identité avec le CYP73A1 d'*Helianthus tuberosus*.

### Exemple 2: Modifications de la séquence codant pour le cytochrome P450 de blé CYP73A17

Contrairement au CYP73A1 d'*Helianthus tuberosus*, lequel peut être exprimé dans les levures (Urban & coll., 1994), des tentatives répétées d'expression de CYP73A17 de blé dans les levures par les même techniques usuelles se sont révélées infructueuses, alors que la séquence nucléotidique n'a pas été modifiée lors de son insertion dans le vecteur d'expression (vérification par séquençage). Aucune protéine n'est détectée par spectrophotométrie ou par immunoblot, de même qu'aucune activité enzymatique n'est détectable dans les microsomes de levure transformée et induite.

### 1. Modification de séquence codante

La séquence codante pour le CYP73A17 de blé (SEQ. ID No 1) a donc été modifiée de trois manières différentes par mutagénèse induite par RPC comme suit:

Les sites de restriction *BamHI* et *EcoRI* ont été respectivement introduits par RPC juste en amont du codon ATG et juste en aval du codon stop de la séquence codante de CYP73A17 (source, origine) en employant les amorces sens et réverse ci-dessous les sites de restrictions étant *BamHI* pour les amorces sens Rec1 (SEQ ID No 3), Rec2 (SEQ ID No 4) et Rec3 (SEQ ID No 5), et *EcoRI* pour l'amorce réverse(SEQ ID No 6).

Une amorce représentée par la SEQ ID No2 a également employée pour permettre la transformation des levures avec la séquence codante pour le CYP73A17 de blé non modifiée (natif).

Les cinq amorces ci-dessus ont été obtenues auprès de la société Eurogentech, synthètisées et purifiées selon les méthodes usuelles.

Pour chaque modification, avec les quatre différentes amorces sens, le mode opératoire est le suivant:

Le mélange réactionnel (20 mM Tris-HCl pH 8,75, 10 mM KCl, 10 mM (NH₄)₂SO₄, 2 mM MgSO₄, 0,1% Triton X100, 0,1 mg/ml BSA, 5 % (v/v) DMSO, 300 µM dNTP, 20 pmoles de chaque amorce, 150 ng template, volume total 50 µl) est préchauffé pendant 2 minutes à 94 °C, avant l'addition de 5 unités d'ADN polymérase Pfu (Stratagène). Après 2 minutes à 94 °C, on procède à 30 cycles d'amplification comme suit: 1 minute de dénaturation à 94 °C, 2 minutes d'hybridation à 55 °C, 2 minutes d'extention à 72 °C. La réaction est complétée par 10 minutes d'extension à 72 °C.

On obtient pour chaque amorce une séquence dérivée de la séquence ID n°1, représentées pour les séquences codantes modifiées par les séquences ID No 7, No 8 et No 9. Les extrémités 5' des séquences obtenues avec les quatre amorces sens ci-dessus sont représentées ci-dessous, le site de restriction *BamHI* étant marqué en italliques:

natif:    ATATATGGATCC ATG GAC GTC CTC CTC CTG GAG AAG GCC
Rec 1     ATATATGGATCC ATG GAT GTT TTG TTG TTG GAG AAG GCC
Rec 2     ATATATGGATCC ATG GAT GTT TTG TTG TTG GAA AAA GCT
Rec 3     ATATATGGATCC ATG GAT GTT TTG TTG TTG GAA AAA GCT
Protéine:              met asp val leu leu leu glu lys ala

CTC CTG GGC CTC TTC GCC GCG GCG GTG CTG GCC ATC GCC GTC GCC
CTC CTG GGC CTC TTC GCC GCG GCG GTG CTG GCC ATC GCC GTC GCC
TTG TTG GGT TTG TTC GCC GCG GCG GTG CTG GCC ATC GCC GTC GCC
TTG TTG GGT TTG TTT GCT GCT GCT GTT TTG GCT ATT GCT GTT GCT
leu leu gly leu phe ala ala ala val leu ala ile ala val ala

AAG CTC ACC GGC AAG CGC TTC CGC CTC CCC CCT GGC CCC TCC GGC
AAG CTC ACC GGC AAG CGC TTC CGC CTC CCC CCT GGC CCC TCC GGC
AAG CTC ACC GGC AAG CGC TTC CGC CTC CCC CCT GGC CCC TCC GGC
AAA TTG ACT GGT AAA AGA TTT AGA TTG CCA CCA GGT CCA TCC GGC
lys leu thr gly lys arg phe arg leu pro pro gly pro ser gly

GCC CCC ATC GTC .....
GCC CCC ATC GTC .....
GCC CCC ATC GTC .....
GCC CCC ATC GTC .....
ala pro ile val .....

### 2. Transformation des levures

Après digestion par les enzymes de restriction *BamHI* et *EcoRI*, les quatre séquences codantes modifiées ci-dessus, sont intégrées dans le vecteur pYeDP60 décrit par Pompon & coll. (*Methods Enzymol*, 272, 1996, 51-64; WO 97/10344), dont le contenu est intégré ici par référence pour ce qui concerne le plasmide, la méthode d'insertion dans le plasmide et la méthode de transformation et de croissance des levures, en particulier à partir des levures *Saccharomyces cerevisiae* souches W(R), WAT21 et WAT11. La méthode de transformation et de croissance des levures est également décrite par Pompon & coll. et par Urban & coll. (*Eur. J Biochem*., 222, 1994, page 844, 2ème colonne, "Yeast transformation and cell culture").

On obtient 4 souches de levures transformées dénommées: W73A17(natif), W73A17(Rec1), W73A17(Rec2) et W73A17(Rec3).

### Exemple 3: Expression de CYP73A17 dans les levures modifiées

Les levures transformées obtenues précédemment sont cultivées selon la méthode décrite par Urban & coll. (*Eur. J. Biochem*., 222, 1994, page 844, 2ème colonne, "Yeast transformation and cell culture") dans 50 ml de milieu SGI pendant 72 h à 30 °C. Les cellules sont récupérées par 10 minutes de centrifugation à 8000 g, lavées avec 25 ml de milieu YPI, recentrifugées, puis remises en suspension dans 250 ml de milieu YPI. L'induction par le galactose est mise en oeuvre pendant 14-16 h sous agitation à 160 rpm, jusqu'à ce que la densité cellulaire atteigne 10⁸ cellules par ml. Les microsomes sont ensuites préparés selon la méthode décrite par Pierrel & coll. (*Eur. J Biochem.*, 224, 1994, 835-844).

L'expression de CYP73A17 pour les quatre souches est quantifiée par spectrophotométrie différentielle selon la méthode décrite par Omura et Sato (*J*. *Biol*. *Chem., 177, 678-693*). Elle est proportionnelle au nombre de codons mal adaptés modifiés.

L'activité enzymatique microsomale est mesurée selon la méthode décrite par Durst F., Benveniste I., Schalk M. and Werck-Reichhart D. (1996) Cinnamic acid hydroxylase activity in plant microsomes. Methods Enzymol. 272, 259-268. Les résultats obtenus après transformation de WAT21 sont reportés sur le Tableau ci-dessous. L'activité est exprimée en activité cinnamate 4-hydroxylase. Le pourcentage additionnel d'activité (valeurs arrondies), permet d'illustrer l'importance du saut d'activité observé après modification des codons mal adaptés.

| Souche | Activité pmol/min/µg protéine | % activité additionnelle |
|---|---|---|
| W73A17 natif | 0,64 | - |
| W73A17 Rec1 | 2,84 | + 340 |
| W73A17 Rec2 | 4,92 | + 670 |
| W73A17 Rec3 | 8,90 | + 1300 |

Ces résultats relatifs à l'augmentation de l'activité enzymatique viennent confirmer ceux relatifs à l'augmentation de l'expression de la protéine dans les levures. Ils montrent qu'une transformation de la seule extrémité en 5', même limitée (Rec 1), est suffisante pour obtenir une amélioration très importante de la production d'enzyme par la levure, et de son activité enzymatique.

### Exemple 4: Expression de CYF86A5 de blé dans les levures modifiées

La séquence codant pour le cytochrome P450 de blé CYP86A5 de blé, représentée par l'identificateur de séquence No 10 (SEQ ID No 10) a été isolée de la banque d'ADNc de blé décrite à l'exemple 1 selon le même mode opératoire décrit pour la séquence de CYP73A17, en employant comme sonde la séquence codante complète de CYP86A1 d'*Arabidopsis thaliana*. Cette séquence de CYP86A5 de blé a été modifiée selon le mode opératoire de l'exemple 2 en employant comme amorces sens et réverse les deux oligonucléotides représentés par les séquences ID No 12 et 13 (SEQ ID No 12 et SEQ ID No 13), respectivement, pour obtenir la séquence codante modifiée selon l'invention représentée par l'identificateur de séquence No. 14 (SEQ ID No 14).

Une amorce représentée par la SEQ ID No 11 a également employée pour permettre la transformation des levures avec la séquence codante pour le CYP86A5 de blé non modifiée (natif).

Les levures sont transformées avec cette nouvelle séquence codante et l'expression quantifiée par spectrophotométrie différentielle selon le mode opératoire de l'exemple 2. Alors que la séquence de CYP86A5 de blé naturelle n'est pas exprimée de façon détectable, la séquence modifiée selon l'invention est exprimée de manière substantielle dans les levures transformées.

Les exemples ci-dessus démontrant sans ambiguïté que pour des séquences d'ADN comprenant en 5, une région à forte teneur en codons mal adaptés aux levures, le simple recodage selon l'invention, même partiel, de cette seule région avec des codons correspondants adaptés aux levures permet une amélioration substantielle de leur expression dans les levures.

## Revendications

1. Séquence d'ADN codant pour une protéine d'intérêt contenant des régions à forte teneur en codons mal adaptés aux levures, caractérisée en ce qu'un nombre suffisant de codons mal adaptés aux levures est remplacé par des codons correspondants adaptés aux levures dans lesdites régions à forte teneur en codons mal adaptés aux levures.

2. Séquence selon la revendication 1, caractérisée en ce que les codons mal adaptés aux levures sont choisis parmi les codons dont la fréquence d'utilisation par les levures est inférieure ou égale à environ 13 pour 1000, de préférence inférieure ou égale à environ 12 pour 1000, plus préférentiellement inférieure ou égale à environ 10 pour 1000.

3. Séquence selon la revendication 2, caractérisée en ce que les codons mal adaptés aux levures sont choisis parmi les codons CTC, CTG et CTT codant pour la leucine, les codons CGG, CGC, CGA, CGT et AGG codant pour l'arginine, les codons GCG et GCC codant pour l'alanine, les codons GGG, GGC et GGA codant pour la glycine et les codons CCG et CCC codant pour la proline.

4. Séquence selon la revendication 3, caractérisée en ce que les codons mal adaptés aux levures sont choisis parmi les codons CTC et CTG codant pour la leucine, les codons CGG, CGC, CGA, CGT et AGG codant pour l'arginine, les codon GCG et GCC pour l'alanine, les codons GGG et GGC codant pour la glycine et les codons CCG et CCC codant pour la proline.

5. Séquence selon l'une des revendications 1 à 4, caractérisée en ce que les codons correspondants adaptés aux levures sont choisis parmi les codons correspondants aux codons mal adaptés aux levures, codant pour les même acides aminés et dont la féquence d'utilisation par les levures est supérieure à 15 pour 1000, de préférence supérieure ou égale à 18 pour 1000, plus préférentiellement supérieure ou égale à 20 pour 1000.

6. Séquence selon la revendication 5, caractérisée en ce que les codons correspondants adaptés aux levures sont choisis parmi les codons TTG et TTA, préférentiellement TTG, codant pour la leucine, le codon AGA codant pour l'arginine, les codons GCT et GCA, préférentiellement GCT, codant pour l'alanine, le codon GGT codant pour la glycine et le codon CCA codant pour la proline.

7. Séquence selon l'une des revendications 1 à 7, caractérisée en ce que les régions à forte teneur en codons mal adaptés aux levures comprennent au moins 2 codons mal adaptés parmi 10 codons consécutifs, les deux codons pouvant être adjacents ou séparées par jusqu'à 8 autres codons.

8. Séquence selon la revendication 7, caractérisée en ce que les régions à forte teneur en codons mal adaptés comprennent 2, 3, 4, 5 ou 6 codons mal adaptés pour 10 codons consécutifs, ou comprennent au moins 2 ou 3 codons mal adaptés adjacents.

9. Séquence d'ADN, en particuler d'ADNc, codant pour une protéine d'intérêt comprenant des régions à forte teneur en leucine, caractérisée en ce qu'un nombre suffisant de codons CTC codant pour la leucine dans ladite région à forte teneur en leucine, est remplacé par des codons TTG et/ou TTA, ou en ce qu'un nombre suffisant de codons CTC et CTG codant pour la leucine dans ladite région à forte teneur en leucine, sont remplacés par des codons TTG et/ou TTA.

10. Séquence selon la revendication 9, caractérisée en ce que les codons CTC ou CTC et CTG sont remplacés par un codon TTG.

11. Séquence selon l'une des revendications 9 ou 10, caractérisée en ce que les régions à forte teneur en leucine comprennent 2, 3, 4, 5 ou 6 leucines pour 10 acides aminés consécutifs, ou comprennent au moins 2 ou 3 leucines adjacentes.

12. Séquence selon l'une des revendications 1 à 11, caractérisée en ce la teneur générale en codons mal adaptés est d'au moins 20 %, plus préférentiellement d'au moins 30 %, par rapport au nombre total de codons.

13. Séquence selon l'une des revendications 1 à 12, caracterisée en ce qu'elle comprend au moins une région en 5' à forte teneur en codons mal adaptés aux levures.

14. Séquence selon la revendication 13, caractérisée en ce que les codons mal adaptés aux levures sont remplacés dans cette seule région en 5'.

15. Séquence selon l'une des revendications 1 à 14, caractérisée en ce qu'il s'agit d'une séquence d'ADN isolée d'origine naturelle, en particulier d'origine végétale.

16. Séquence selon la revendication 15, caractérisée en ce quelle est d'origine de plantes dicotylédones ou monocotyledones, en particulier de plantes monocotyledones.

17. Séquence selon la revendication 16, caractérisée en ce qu'elle est d'origine de plantes de la famille des graminées, en particulier choisies parmi le blé, l'orge, l'avoine, le riz, le maïs, le sorgho ou la canne à sucre.

18. Séquence selon l'une des revendications 1 à 17, caractérisée en ce qu'elle code pour une enzyme.

19. Séquence selon la revendication 18, caractérisée en ce qu'elle code pour un cytochrome P450.

20. Séquence selon la revendication 19, caractérisée en ce que la séquence contenant des régions à forte teneur en codons mal adaptés aux levures comprend la région codante des séquences ID No 1 ou ID No 10.

21. Séquence selon la revendication 19, caractérisée en ce qu'il s'agit des séquences ID No 7, ID No 8, ID No 9 ou ID No 13.

22. Gène chimère comprenant une séquence d'ADN modifiée selon l'une des revendications 1 à 21 et des éléments de régulation en position 5' et 3' hétérologues pouvant fonctionner dans une levure.

23. Vecteur pour la transformation des levures comprenant au moins un gène chimère selon la revendication 22.

24. Procédé de transformation des levures au moyen d'un vecteur selon la revendication 23.

25. Levure transformée pour l'expression d'une protéine d'intérrêt caractérisée en ce qu'elle comprend un gène chimère selon la revendication 22.

26. Levure selon la revendication 25, caractérisée en ce qu'elle est choisie parmi les genres *Saccharomyces, Kluyveromyces, Hansenula, Pichia* et *Yarrowia,* avantageusement du genre *Saccharomyces*, en particulier *S. cerevisiae*.

27. Procédé de production d'une protéine d'intérêt hétérologue dans une levure transformée, caractérisée en ce qu'elle comprend les étapes de:
a) transformation d'une levure avec un vecteur selon la revendication 23, comprenant une séquence d'ADN modifiée selon l'une des revendications 1 à 21 et des éléments de régulation en position 5' et 3' hétérologues pouvant fonctionner dans une levure,
b) culture de la levure transformée, et
c) extraction de la protéine d'intérêt de la culture de levure.

28. Procédé de transformation d'un substrat par catalyse enzymatique au moyen d'une enzyme exprimée dans une levure, comprenant les étapes de
a) culture en présence du substrat à transformer, de la levure transformée avec un vecteur selon la revendication 23, comprenant une séquence d'ADN modifiée selon l'une des revendications 1 à 21 et des éléments de régulation en position 5' et 3' hétérologues pouvant fonctionner dans une levure, puis
b) récupération du substrat transformé de la culture de levure.


LISTE DE SEQUENCES

(1) INFORMATIONS GENERALES:

(iii) NOMBRE DE SEQUENCES: 14

(2) INFORMATIONS POUR LA SEQ ID NO: 1:

(i) CARACTERISTIQUES DE LA SEQUENCE:
(A) LONGUEUR: 2261 paires de bases
(B) TYPE: nucléotide
(C) NOMBRE DE BRINS: simple
(D) CONFIGURATION: linéaire

(ii) TYPE DE MOLECULE: ADNc

(ix) CARACTERISTIQUE:
(A) NOM/CLE: CDS
(B) EMPLACEMENT:49..1551

(xi) DESCRIPTION DE LA SEQUENCE: SEQ ID NO: 1:

CGCAGCACGG CAACACATAC ACAGGAGCCA CACACCGCAC CTACCCCG ATG GAC GTC 57
Met Asp Val
1

CTC CTC CTG GAG AAG GCC CTC CTG GGC CTC TTC GCC GCG GCG GTG CTG 105
Leu Leu Leu Glu Lys Ala Leu Leu Gly Leu Phe Ala Ala Ala Val Leu
    5                   10                  15

GCC ATC GCC GTC GCC AAG CTC ACC GGC AAG CGC TTC CGC CTC CCC CCT 153
Ala Ile Ala Val Ala Lys Leu Thr Gly Lys Arg Phe Arg Leu Pro Pro
20                  25                  30                  35

GGC CCC TCC GGC GCC CCC ATC GTC GGC AAC TGG CTG CAG GTC GGC GAC 201
Gly Pro Ser Gly Ala Pro Ile Val Gly Asn Trp Leu Gln Val Gly Asp
                40                  45                  50

GAC CTC AAC CAC CGC AAC CTG ATG GGC CTG GCC AAG CGG TTC GGC GAG 249
Asp Leu Asn His Arg Asn Leu Met Gly Leu Ala Lys Arg Phe Gly Glu
            55                  60                  65

GTG TTC CTC CTC CGC ATG GGC GTC CGC AAC CTG GTG GTC GTC TCC AGC 297
Val Phe Leu Leu Arg Met Gly Val Arg Asn Leu Val Val Val Ser Ser
        70                  75                  80

CCC GAG CTC GCC AAG GAG GTC CTC CAC ACC CAG GGC GTC GAG TTC GGC 345
Pro Glu Leu Ala Lys Glu Val Leu His Thr Gln Gly Val Glu Phe Gly
    85                  90                  95

TCC CGC ACC CGC AAC GTC GTC TTC GAC ATC TTC ACC GGC AAG GGA CAG 393
Ser Arg Thr Arg Asn Val Val Phe Asp Ile Phe Thr Gly Lys Gly Gln
100                 105                 110                 115

GAC ATG GTG TTC ACG GTG TAC GGC GAC CAC TGG CGC AAG ATG CGG CGG 441
Asp Met Val Phe Thr Val Tyr Gly Asp His Trp Arg Lys Met Arg Arg
                120                 125                 130

ATC ATG ACG GTG CCC TTC TTC ACC AAC AAG GTG GTG GCG CAG AAC CGC 489
Ile Met Thr Val Pro Phe Phe Thr Asn Lys Val Val Ala Gln Asn Arg
            135                 140                 145

GTG GGG TGG GAG GAG GAG GCC CGG CTG GTG GTG GAG GAC CTC AAG GCC 537
Val Gly Trp Glu Glu Glu Ala Arg Leu Val Val Glu Asp Leu Lys Ala
        150                 155                 160

GAC CCG GCG GCG GCG ACG GCG GGC GTG GTG GTC CGC CGC AGG CTG CAG 585
Asp Pro Ala Ala Ala Thr Ala Gly Val Val Val Arg Arg Arg Leu Gln
    165                 170                 175

CTC ATG ATG TAC AAC GAC ATG TTC CGC ATC ATG TTC GAC CGC CGG TTC 633
Leu Met Met Tyr Asn Asp Met Phe Arg Ile Met Phe Asp Arg Arg Phe
180                 185                 190                 195

GAG AGC GTG GCC GAC CCG CTC TTC AAC CAG CTC AAG GCG CTC AAC GCC 681
Glu Ser Val Ala Asp Pro Leu Phe Asn Gln Leu Lys Ala Leu Asn Ala
                200                 205                 210

GAG CGC AGC ATC CTC TCC CAG AGC TTC GAC TAC AAC TAC GGC GAC TTC 729
Glu Arg Ser Ile Leu Ser Gln Ser Phe Asp Tyr Asn Tyr Gly Asp Phe
            215                 220                 225

ATC CCC GTC CTC CGC CCC TTC CTC CGC CGC TAC CTC AAC CGC TGC ACC 777
Ile Pro Val Leu Arg Pro Phe Leu Arg Arg Tyr Leu Asn Arg Cys Thr
        230                 235                 240

AAC CTC AAG ACC AAG CGG ATG AAG GTG TTC GAG GAC CAC TTC GTC CAG 825
Asn Leu Lys Thr Lys Arg Met Lys Val Phe Glu Asp His Phe Val Gln
    245                 250                 255

CAG CGC AAG GAG GCG TTG GAG AAG ACG GGT GAG ATC AGG TGC GCC ATG 873
Gln Arg Lys Glu Ala Leu Glu Lys Thr Gly Glu Ile Arg Cys Ala Met
260                 265                 270                 275

GAC CAC ATC CTG GAA GCC GAA AGG AAG GGC GAG ATC AAC CAC GAC AAC 921
Asp His Ile Leu Glu Ala Glu Arg Lys Gly Glu Ile Asn His Asp Asn
                280                 285                 290

GTC CTC TAC ATC GTC GAG AAC ATC AAC GTC GCA GCC ATC GAG ACG ACG 969
Val Leu Tyr Ile Val Glu Asn Ile Asn Val Ala Ala Ile Glu Thr Thr
            295                 300                 305

CTG TGG TCG ATC GAG TGG GGC CTC GCG GAG CTG GTG AAC CAC CCG GAG 1017
Leu Trp Ser Ile Glu Trp Gly Leu Ala Glu Leu Val Asn His Pro Glu
        310                 315                 320

ATC CAG CAG AAG CTG CGC GAG GAG ATC GTC GCC GTT CTG GGC GCC GGC 1065
Ile Gln Gln Lys Leu Arg Glu Glu Ile Val Ala Val Leu Gly Ala Gly
    325                 330                 335

GTG GCG GTG ACG GAG CCG GAC CTG GAG CGC CTC CCC TAC CTG CAG TCC 1113
Val Ala Val Thr Glu Pro Asp Leu Glu Arg Leu Pro Tyr Leu Gln Ser
340                 345                 350                 355

GTG GTG AAG GAG ACG CTC CGC CTC CGC ATG GCA ATC CCG CTC CTG GTG 1161
Val Val Lys Glu Thr Leu Arg Leu Arg Met Ala Ile Pro Leu Leu Val
                360                 365                 370

CCG CAC ATG AAC CTC AGC GAC GCC AAG CTC GCC GGC TAC GAC ATC CCC 1209
Pro His Met Asn Leu Ser Asp Ala Lys Leu Ala Gly Tyr Asp Ile Pro
            375                 380                 385

GCC GAG TCC AAG ATC CTC GTC AAC GCC TGG TTC CTC GCC AAC GAC CCC 1257
Ala Glu Ser Lys Ile Leu Val Asn Ala Trp Phe Leu Ala Asn Asp Pro
        390                 395                 400

AAG CGG TGG GTG CGC GCC GAT GAG TTC AGG CCG GAG AGG TTC CTC GAG 1305
Lys Arg Trp Val Arg Ala Asp Glu Phe Arg Pro Glu Arg Phe Leu Glu
    405                 410                 415

GAG GAG AAG GCC GTC GAG GCC CAC GGC AAC GAT TTC CGG TTC GTG CCC 1353
Glu Glu Lys Ala Val Glu Ala His Gly Asn Asp Phe Arg Phe Val Pro
420                 425                 430                 435

TTC GGC GTC GGC CGC CGG AGC TGC CCC GGG ATC ATC CTC GCG CTG CCC     1401
Phe Gly Val Gly Arg Arg Ser Cys Pro Gly Ile Ile Leu Ala Leu Pro
                440                 445                 450

ATC ATC GGC ATC ACG CTC GGA CGC CTG GTG CAG AAC TTC CAG CTG CTG     1449
Ile Ile Gly Ile Thr Leu Gly Arg Leu Val Gln Asn Phe Gln Leu Leu
            455                 460                 465

CCG CCG CCG GGG CAG GAC AAG ATC GAC ACC ACC GAG AAG CCC GGG CAG     1497
Pro Pro Pro Gly Gln Asp Lys Ile Asp Thr Thr Glu Lys Pro Gly Gln
        470                 475                 480

TTT ACC AAC CAG ATC CTC AAG CAC GCC ACC ATT GTC TGC AAG CCA CTC     1545
Phe Thr Asn Gln Ile Leu Lys His Ala Thr Ile Val Cys Lys Pro Leu
    485                 490                 495

GAG GCT TAACTGAATT GAGGTTTCGG TCATGGGCGC CCGCTGACGC GGGGAGATGG     1601
Glu Ala
500

ATCTATGCAT GTGACTGTGT ATTTTGCCTT CTTTCTTTTT GGTGTTGTTT TTTGCAGTAG     1661

TAAGTTTAAT TTTTCTTTGG TGTTGGCCTA TTTGTCTTCA TGTGAGGCGT CGTGTTGTAA     1721

ATTTCCATAT AGTTGGCAAT GTGATGTAAA ACTTGGCTCC AAAAAAAAAA AAAAAAAACT     1781

CGAGACTCTT CTCTCTCTCT CTCTCTCTCC AGCCTCGGGT CTCTGCTGGC AAGGGAACTT     1841

GCATTACCCT GTGTACGACG GCGCCATGTT CGTCCCTGAA GCACCCTCCC TGCAGAGCTC     1901

CCAGGACAAC TTCGCTGCAT CTGCTGGTTT CAAGCGTCGA AGGAGAGAGT TTTGAATACC     1961

CGAAAGAATA TAGCGTTGGA CATATCTGTC AAACAGGGGA TCTTGCTGTG GGTCTCTTGG     2021

TGGGCCAAAT CGCATAGACA ATCATTCAAA TGGATGGGTT CTTCGCTGGT CGGTCAAAAA     2081

GTATATGTTG TAATTGTACG CCTTTTTTGG GTCTTGTTGC CAAAGATCAT GGTTATTGAG     2141

TTGTGAGCTC TGAGATAACA GGTTTGTGTA TAGTGAAATA AAGAGGAGCG TCGTCAACAC     2201

CATGTACTAT ATAGGCTTTG AAATTCCATT AAGATGCATC AGAAATCAAT GTTGGATTTG     2261

(2) INFORMATIONS POUR LA SEQ ID NO: 2:

     (i) CARACTERISTIQUES DE LA SEQUENCE:
          (A) LONGUEUR: 38 paires de bases
          (B) TYPE: nucléotide
          (C) NOMBRE DE BRINS: simple
          (D) CONFIGURATION: linéaire

    (ii) TYPE DE MOLECULE: Autre acide nucléique
          (A) DESCRIPTION: /desc = "Amorce"

(xi) DESCRIPTION DE LA SEQUENCE: SEQ ID NO: 2:

ATATATGGAT CCATGGACGT CCTCCTCCTG GAGAAGGC                              38

(2) INFORMATIONS POUR LA SEQ ID NO: 3:

     (i) CARACTERISTIQUES DE LA SEQUENCE:
          (A) LONGUEUR: 56 paires de bases
          (B) TYPE: nucléotide
          (C) NOMBRE DE BRINS: simple
          (D) CONFIGURATION: linéaire

    (ii) TYPE DE MOLECULE: Autre acide nucléique

(A) DESCRIPTION: /desc = "Amorce"

(xi) DESCRIPTION DE LA SEQUENCE: SEQ ID NO: 3:

ATATATGGAT CCATGGATGT TTTGTTGTTG GAGAAGGCCC TCCTGGGCCT CTTCGC 56

(2) INFORMATIONS POUR LA SEQ ID NO: 4:

(i) CARACTERISTIQUES DE LA SEQUENCE:
(A) LONGUEUR: 71 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "Amorce"

(xi) DESCRIPTION DE LA SEQUENCE: SEQ ID NO: 4:

ATATATGGAT CCATGGATGT TTTGTTGTTG GAAAAAGCTT TGTTGGGTTT GTTCGCCGCG 60

GCGGTGCTGG C 71

(2) INFORMATIONS POUR LA SEQ ID NO: 5:

(i) CARACTERISTIQUES DE LA SEQUENCE:
(A) LONGUEUR: 143 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "Amorce"

(xi) DESCRIPTION DE LA SEQUENCE: SEQ ID NO: 5:

ATATATGGAT CCATGGATGT TTTGTTGTTG GAAAAAGCTT TGTTGGGTTT GTTTGCTGCT 60

GCTGTTTTGG CTATTGCTGT TGCTAAATTG ACTGGTAAAA GATTTAGATT GCCACCAGGT 120

CCATCCGGCG CCCCCATCGT CGG 143

(2) INFORMATIONS POUR LA SEQ ID NO: 6:

(i) CARACTERISTIQUES DE LA SEQUENCE:
(A) LONGUEUR: 39 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "Reverse"

(xi) DESCRIPTION DE LA SEQUENCE: SEQ ID NO: 6:

TATATAGAAT TCCAGTTAAG CCTCGAGTGG CTTGCAGAC 39

(2) INFORMATIONS POUR LA SEQ ID NO: 7:

(i) CARACTERISTIQUES DE LA SEQUENCE:
(A) LONGUEUR: 1506 paires de bases
(B) TYPE: nucléotide
(C) NOMBRE DE BRINS: simple
(D) CONFIGURATION: linéaire

(ii) TYPE DE MOLECULE: ADNc

(ix) CARACTERISTIQUE:
(A) NOM/CLE: CDS
(B) EMPLACEMENT:1..1503

(xi) DESCRIPTION DE LA SEQUENCE: SEQ ID NO: 7:

ATG GAT GTT TTG TTG TTG GAG AAG GCC CTC CTG GGC CTC TTC GCC GCG 48
Met Asp Val Leu Leu Leu Glu Lys Ala Leu Leu Gly Leu Phe Ala Ala
1               5                   10                  15

GCG GTG CTG GCC ATC GCC GTC GCC AAG CTC ACC GGC AAG CGC TTC CGC 96
Ala Val Leu Ala Ile Ala Val Ala Lys Leu Thr Gly Lys Arg Phe Arg
            20                  25                  30

CTC CCC CCT GGC CCC TCC GGC GCC CCC ATC GTC GGC AAC TGG CTG CAG 144
Leu Pro Pro Gly Pro Ser Gly Ala Pro Ile Val Gly Asn Trp Leu Gln
        35                  40                  45

GTC GGC GAC GAC CTC AAC CAC CGC AAC CTG ATG GGC CTG GCC AAG CGG 192
Val Gly Asp Asp Leu Asn His Arg Asn Leu Met Gly Leu Ala Lys Arg
    50                  55                  60

TTC GGC GAG GTG TTC CTC CTC CGC ATG GGC GTC CGC AAC CTG GTG GTC 240
Phe Gly Glu Val Phe Leu Leu Arg Met Gly Val Arg Asn Leu Val Val
65                  70                  75                  80

GTC TCC AGC CCC GAG CTC GCC AAG GAG GTC CTC CAC ACC CAG GGC GTC 288
Val Ser Ser Pro Glu Leu Ala Lys Glu Val Leu His Thr Gln Gly Val
                85                  90                  95

GAG TTC GGC TCC CGC ACC CGC AAC GTC GTC TTC GAC ATC TTC ACC GGC 336
Glu Phe Gly Ser Arg Thr Arg Asn Val Val Phe Asp Ile Phe Thr Gly
            100                 105                 110

AAG GGA CAG GAC ATG GTG TTC ACG GTG TAC GGC GAC CAC TGG CGC AAG 384
Lys Gly Gln Asp Met Val Phe Thr Val Tyr Gly Asp His Trp Arg Lys
        115                 120                 125

ATG CGG CGG ATC ATG ACG GTG CCC TTC TTC ACC AAC AAG GTG GTG GCG 432
Met Arg Arg Ile Met Thr Val Pro Phe Phe Thr Asn Lys Val Val Ala
    130                 135                 140

CAG AAC CGC GTG GGG TGG GAG GAG GAG GCC CGG CTG GTG GTG GAG GAC 480
Gln Asn Arg Val Gly Trp Glu Glu Glu Ala Arg Leu Val Val Glu Asp
145                 150                 155                 160

CTC AAG GCC GAC CCG GCG GCG GCG ACG GCG GGC GTG GTG GTC CGC CGC 528
Leu Lys Ala Asp Pro Ala Ala Ala Thr Ala Gly Val Val Val Arg Arg
                165                 170                 175

AGG CTG CAG CTC ATG ATG TAC AAC GAC ATG TTC CGC ATC ATG TTC GAC 576
Arg Leu Gln Leu Met Met Tyr Asn Asp Met Phe Arg Ile Met Phe Asp
            180                 185                 190

CGC CGG TTC GAG AGC GTG GCC GAC CCG CTC TTC AAC CAG CTC AAG GCG 624
Arg Arg Phe Glu Ser Val Ala Asp Pro Leu Phe Asn Gln Leu Lys Ala
        195                 200                 205

CTC AAC GCC GAG CGC AGC ATC CTC TCC CAG AGC TTC GAC TAC AAC TAC 672
Leu Asn Ala Glu Arg Ser Ile Leu Ser Gln Ser Phe Asp Tyr Asn Tyr
    210                 215                 220

GGC GAC TTC ATC CCC GTC CTC CGC CCC TTC CTC CGC CGC TAC CTC AAC 720
Gly Asp Phe Ile Pro Val Leu Arg Pro Phe Leu Arg Arg Tyr Leu Asn
225                 230                 235                 240

CGC TGC ACC AAC CTC AAG ACC AAG CGG ATG AAG GTG TTC GAG GAC CAC 768
Arg Cys Thr Asn Leu Lys Thr Lys Arg Met Lys Val Phe Glu Asp His
                245                 250                 255

TTC GTC CAG CAG CGC AAG GAG GCG TTG GAG AAG ACG GGT GAG ATC AGG 816
Phe Val Gln Gln Arg Lys Glu Ala Leu Glu Lys Thr Gly Glu Ile Arg
            260                 265                 270

TGC GCC ATG GAC CAC ATC CTG GAA GCC GAA AGG AAG GGC GAG ATC AAC 864
Cys Ala Met Asp His Ile Leu Glu Ala Glu Arg Lys Gly Glu Ile Asn
        275                 280                 285

CAC GAC AAC GTC CTC TAC ATC GTC GAG AAC ATC AAC GTC GCA GCC ATC 912
His Asp Asn Val Leu Tyr Ile Val Glu Asn Ile Asn Val Ala Ala Ile
    290                 295                 300

GAG ACG ACG CTG TGG TCG ATC GAG TGG GGC CTC GCG GAG CTG GTG AAC 960
Glu Thr Thr Leu Trp Ser Ile Glu Trp Gly Leu Ala Glu Leu Val Asn
305                 310                 315                 320

CAC CCG GAG ATC CAG CAG AAG CTG CGC GAG GAG ATC GTC GCC GTT CTG 1008
His Pro Glu Ile Gln Gln Lys Leu Arg Glu Glu Ile Val Ala Val Leu
                325                 330                 335

GGC GCC GGC GTG GCG GTG ACG GAG CCG GAC CTG GAG CGC CTC CCC TAC 1056
Gly Ala Gly Val Ala Val Thr Glu Pro Asp Leu Glu Arg Leu Pro Tyr
            340                 345                 350

CTG CAG TCC GTG GTG AAG GAG ACG CTC CGC CTC CGC ATG GCA ATC CCG 1104
Leu Gln Ser Val Val Lys Glu Thr Leu Arg Leu Arg Met Ala Ile Pro
        355                 360                 365

CTC CTG GTG CCG CAC ATG AAC CTC AGC GAC GCC AAG CTC GCC GGC TAC 1152
Leu Leu Val Pro His Met Asn Leu Ser Asp Ala Lys Leu Ala Gly Tyr
    370                 375                 380

GAC ATC CCC GCC GAG TCC AAG ATC CTC GTC AAC GCC TGG TTC CTC GCC 1200
Asp Ile Pro Ala Glu Ser Lys Ile Leu Val Asn Ala Trp Phe Leu Ala
385                 390                 395                 400

AAC GAC CCC AAG CGG TGG GTG CGC GCC GAT GAG TTC AGG CCG GAG AGG 1248
Asn Asp Pro Lys Arg Trp Val Arg Ala Asp Glu Phe Arg Pro Glu Arg
                405                 410                 415

TTC CTC GAG GAG GAG AAG GCC GTC GAG GCC CAC GGC AAC GAT TTC CGG 1296
Phe Leu Glu Glu Glu Lys Ala Val Glu Ala His Gly Asn Asp Phe Arg
            420                 425                 430

TTC GTG CCC TTC GGC GTC GGC CGC CGG AGC TGC CCC GGG ATC ATC CTC 1344
Phe Val Pro Phe Gly Val Gly Arg Arg Ser Cys Pro Gly Ile Ile Leu
        435                 440                 445

GCG CTG CCC ATC ATC GGC ATC ACG CTC GGA CGC CTG GTG CAG AAC TTC 1392
Ala Leu Pro Ile Ile Gly Ile Thr Leu Gly Arg Leu Val Gln Asn Phe
    450                 455                 460

CAG CTG CTG CCG CCG CCG GGG CAG GAC AAG ATC GAC ACC ACC GAG AAG 1440
Gln Leu Leu Pro Pro Pro Gly Gln Asp Lys Ile Asp Thr Thr Glu Lys
465                 470                 475                 480

CCC GGG CAG TTT ACC AAC CAG ATC CTC AAG CAC GCC ACC ATT GTC TGC 1488
Pro Gly Gln Phe Thr Asn Gln Ile Leu Lys His Ala Thr Ile Val Cys
                485                 490                 495

AAG CCA CTC GAG GCT TAA 1506
Lys Pro Leu Glu Ala
            500

(2) INFORMATIONS POUR LA SEQ ID NO: 8:

(i) CARACTERISTIQUES DE LA SEQUENCE:
(A) LONGUEUR: 1506 paires de bases
(B) TYPE: nucléotide
(C) NOMBRE DE BRINS: simple
(D) CONFIGURATION: linéaire

(ii) TYPE DE MOLECULE: ADNc

(ix) CARACTERISTIQUE:
(A) NOM/CLE: CDS
(B) EMPLACEMENT:1..1503

(xi) DESCRIPTION DE LA SEQUENCE: SEQ ID NO: 8:

ATG GAT GTT TTG TTG TTG GAA AAA GCT TTG TTG GGT TTG TTC GCC GCG 48
Met Asp Val Leu Leu Leu Glu Lys Ala Leu Leu Gly Leu Phe Ala Ala
1               5                   10                  15

GCG GTG CTG GCC ATC GCC GTC GCC AAG CTC ACC GGC AAG CGC TTC CGC 96
Ala Val Leu Ala Ile Ala Val Ala Lys Leu Thr Gly Lys Arg Phe Arg
            20                  25                  30

CTC CCC CCT GGC CCC TCC GGC GCC CCC ATC GTC GGC AAC TGG CTG CAG 144
Leu Pro Pro Gly Pro Ser Gly Ala Pro Ile Val Gly Asn Trp Leu Gln
        35                  40                  45

GTC GGC GAC GAC CTC AAC CAC CGC AAC CTG ATG GGC CTG GCC AAG CGG 192
Val Gly Asp Asp Leu Asn His Arg Asn Leu Met Gly Leu Ala Lys Arg
    50                  55                  60

TTC GGC GAG GTG TTC CTC CTC CGC ATG GGC GTC CGC AAC CTG GTG GTC 240
Phe Gly Glu Val Phe Leu Leu Arg Met Gly Val Arg Asn Leu Val Val
65                  70                  75                  80

GTC TCC AGC CCC GAG CTC GCC AAG GAG GTC CTC CAC ACC CAG GGC GTC 288
Val Ser Ser Pro Glu Leu Ala Lys Glu Val Leu His Thr Gln Gly Val
                85                  90                  95

GAG TTC GGC TCC CGC ACC CGC AAC GTC GTC TTC GAC ATC TTC ACC GGC 336
Glu Phe Gly Ser Arg Thr Arg Asn Val Val Phe Asp Ile Phe Thr Gly
            100                 105                 110

AAG GGA CAG GAC ATG GTG TTC ACG GTG TAC GGC GAC CAC TGG CGC AAG 384
Lys Gly Gln Asp Met Val Phe Thr Val Tyr Gly Asp His Trp Arg Lys
        115                 120                 125

ATG CGG CGG ATC ATG ACG GTG CCC TTC TTC ACC AAC AAG GTG GTG GCG 432
Met Arg Arg Ile Met Thr Val Pro Phe Phe Thr Asn Lys Val Val Ala
    130                 135                 140

CAG AAC CGC GTG GGG TGG GAG GAG GAG GCC CGG CTG GTG GTG GAG GAC 480
Gln Asn Arg Val Gly Trp Glu Glu Glu Ala Arg Leu Val Val Glu Asp
145                 150                 155                 160

CTC AAG GCC GAC CCG GCG GCG GCG ACG GCG GGC GTG GTG GTC CGC CGC 528
Leu Lys Ala Asp Pro Ala Ala Ala Thr Ala Gly Val Val Val Arg Arg
                165                 170                 175

AGG CTG CAG CTC ATG ATG TAC AAC GAC ATG TTC CGC ATC ATG TTC GAC 576
Arg Leu Gln Leu Met Met Tyr Asn Asp Met Phe Arg Ile Met Phe Asp
            180                 185                 190

CGC CGG TTC GAG AGC GTG GCC GAC CCG CTC TTC AAC CAG CTC AAG GCG 624
Arg Arg Phe Glu Ser Val Ala Asp Pro Leu Phe Asn Gln Leu Lys Ala
        195                 200                 205

CTC AAC GCC GAG CGC AGC ATC CTC TCC CAG AGC TTC GAC TAC AAC TAC 672
Leu Asn Ala Glu Arg Ser Ile Leu Ser Gln Ser Phe Asp Tyr Asn Tyr
    210                 215                 220

GGC GAC TTC ATC CCC GTC CTC CGC CCC TTC CTC CGC CGC TAC CTC AAC 720
Gly Asp Phe Ile Pro Val Leu Arg Pro Phe Leu Arg Arg Tyr Leu Asn
225                 230                 235                 240

CGC TGC ACC AAC CTC AAG ACC AAG CGG ATG AAG GTG TTC GAG GAC CAC 768
Arg Cys Thr Asn Leu Lys Thr Lys Arg Met Lys Val Phe Glu Asp His
                245                 250                 255

TTC GTC CAG CAG CGC AAG GAG GCG TTG GAG AAG ACG GGT GAG ATC AGG 816
Phe Val Gln Gln Arg Lys Glu Ala Leu Glu Lys Thr Gly Glu Ile Arg
            260                 265                 270

TGC GCC ATG GAC CAC ATC CTG GAA GCC GAA AGG AAG GGC GAG ATC AAC 864
Cys Ala Met Asp His Ile Leu Glu Ala Glu Arg Lys Gly Glu Ile Asn
        275                 280                 285

CAC GAC AAC GTC CTC TAC ATC GTC GAG AAC ATC AAC GTC GCA GCC ATC 912
His Asp Asn Val Leu Tyr Ile Val Glu Asn Ile Asn Val Ala Ala Ile
    290                 295                 300

GAG ACG ACG CTG TGG TCG ATC GAG TGG GGC CTC GCG GAG CTG GTG AAC 960
Glu Thr Thr Leu Trp Ser Ile Glu Trp Gly Leu Ala Glu Leu Val Asn
305                 310                 315                 320

CAC CCG GAG ATC CAG CAG AAG CTG CGC GAG GAG ATC GTC GCC GTT CTG 1008
His Pro Glu Ile Gln Gln Lys Leu Arg Glu Glu Ile Val Ala Val Leu
                325                 330                 335

GGC GCC GGC GTG GCG GTG ACG GAG CCG GAC CTG GAG CGC CTC CCC TAC 1056
Gly Ala Gly Val Ala Val Thr Glu Pro Asp Leu Glu Arg Leu Pro Tyr
            340                 345                 350

CTG CAG TCC GTG GTG AAG GAG ACG CTC CGC CTC CGC ATG GCA ATC CCG 1104
Leu Gln Ser Val Val Lys Glu Thr Leu Arg Leu Arg Met Ala Ile Pro
        355                 360                 365

CTC CTG GTG CCG CAC ATG AAC CTC AGC GAC GCC AAG CTC GCC GGC TAC 1152
Leu Leu Val Pro His Met Asn Leu Ser Asp Ala Lys Leu Ala Gly Tyr
    370                 375                 380

GAC ATC CCC GCC GAG TCC AAG ATC CTC GTC AAC GCC TGG TTC CTC GCC 1200
Asp Ile Pro Ala Glu Ser Lys Ile Leu Val Asn Ala Trp Phe Leu Ala
385                 390                 395                 400

AAC GAC CCC AAG CGG TGG GTG CGC GCC GAT GAG TTC AGG CCG GAG AGG 1248
Asn Asp Pro Lys Arg Trp Val Arg Ala Asp Glu Phe Arg Pro Glu Arg
                405                 410                 415

TTC CTC GAG GAG GAG AAG GCC GTC GAG GCC CAC GGC AAC GAT TTC CGG 1296
Phe Leu Glu Glu Glu Lys Ala Val Glu Ala His Gly Asn Asp Phe Arg
            420                 425                 430

TTC GTG CCC TTC GGC GTC GGC CGC CGG AGC TGC CCC GGG ATC ATC CTC 1344
Phe Val Pro Phe Gly Val Gly Arg Arg Ser Cys Pro Gly Ile Ile Leu
        435                 440                 445

GCG CTG CCC ATC ATC GGC ATC ACG CTC GGA CGC CTG GTG CAG AAC TTC 1392
Ala Leu Pro Ile Ile Gly Ile Thr Leu Gly Arg Leu Val Gln Asn Phe
    450                 455                 460

CAG CTG CTG CCG CCG CCG GGG CAG GAC AAG ATC GAC ACC ACC GAG AAG 1440
Gln Leu Leu Pro Pro Pro Gly Gln Asp Lys Ile Asp Thr Thr Glu Lys
465                 470                 475                 480

CCC GGG CAG TTT ACC AAC CAG ATC CTC AAG CAC GCC ACC ATT GTC TGC 1488
Pro Gly Gln Phe Thr Asn Gln Ile Leu Lys His Ala Thr Ile Val Cys
                485                 490                 495

AAG CCA CTC GAG GCT TAA 1506
Lys Pro Leu Glu Ala
            500

(2) INFORMATIONS POUR LA SEQ ID NO: 9:

(i) CARACTERISTIQUES DE LA SEQUENCE:
(A) LONGUEUR: 1506 paires de bases
(B) TYPE: nucléotide
(C) NOMBRE DE BRINS: simple
(D) CONFIGURATION: linéaire

(ii) TYPE DE MOLECULE: ADNc

(ix) CARACTERISTIQUE:
(A) NOM/CLE: CDS
(B) EMPLACEMENT:1..1503

(xi) DESCRIPTION DE LA SEQUENCE: SEQ ID NO: 9:

ATG GAT GTT TTG TTG TTG GAA AAA GCT TTG TTG GGT TTG TTT GCT GCT 48
Met Asp Val Leu Leu Leu Glu Lys Ala Leu Leu Gly Leu Phe Ala Ala
505 510 515

GCT GTT TTG GCT ATT GCT GTT GCT AAA TTG ACT GGT AAA AGA TTT AGA 96
Ala Val Leu Ala Ile Ala Val Ala Lys Leu Thr Gly Lys Arg Phe Arg
520 525 530

TTG CCA CCA GGT CCA TCC GGC GCC CCC ATC GTC GGC AAC TGG CTG CAG 144
Leu Pro Pro Gly Pro Ser Gly Ala Pro Ile Val Gly Asn Trp Leu Gln
        35                  40                  45

GTC GGC GAC GAC CTC AAC CAC CGC AAC CTG ATG GGC CTG GCC AAG CGG 192
Val Gly Asp Asp Leu Asn His Arg Asn Leu Met Gly Leu Ala Lys Arg
    50                  55                  60

TTC GGC GAG GTG TTC CTC CTC CGC ATG GGC GTC CGC AAC CTG GTG GTC 240
Phe Gly Glu Val Phe Leu Leu Arg Met Gly Val Arg Asn Leu Val Val
65                  70                  75                  80

GTC TCC AGC CCC GAG CTC GCC AAG GAG GTC CTC CAC ACC CAG GGC GTC 288
Val Ser Ser Pro Glu Leu Ala Lys Glu Val Leu His Thr Gln Gly Val
                85                  90                  95

GAG TTC GGC TCC CGC ACC CGC AAC GTC GTC TTC GAC ATC TTC ACC GGC 336
Glu Phe Gly Ser Arg Thr Arg Asn Val Val Phe Asp Ile Phe Thr Gly
            100                 105                 110

AAG GGA CAG GAC ATG GTG TTC ACG GTG TAC GGC GAC CAC TGG CGC AAG 384
Lys Gly Gln Asp Met Val Phe Thr Val Tyr Gly Asp His Trp Arg Lys
        115                 120                 125

ATG CGG CGG ATC ATG ACG GTG CCC TTC TTC ACC AAC AAG GTG GTG GCG 432
Met Arg Arg Ile Met Thr Val Pro Phe Phe Thr Asn Lys Val Val Ala
    130                 135                 140

CAG AAC CGC GTG GGG TGG GAG GAG GAG GCC CGG CTG GTG GTG GAG GAC 480
Gln Asn Arg Val Gly Trp Glu Glu Glu Ala Arg Leu Val Val Glu Asp
145                 150                 155                 160

CTC AAG GCC GAC CCG GCG GCG GCG ACG GCG GGC GTG GTG GTC CGC CGC 528
Leu Lys Ala Asp Pro Ala Ala Ala Thr Ala Gly Val Val Val Arg Arg
                165                 170                 175

AGG CTG CAG CTC ATG ATG TAC AAC GAC ATG TTC CGC ATC ATG TTC GAC 576
Arg Leu Gln Leu Met Met Tyr Asn Asp Met Phe Arg Ile Met Phe Asp
            180                 185                 190

CGC CGG TTC GAG AGC GTG GCC GAC CCG CTC TTC AAC CAG CTC AAG GCG 624
Arg Arg Phe Glu Ser Val Ala Asp Pro Leu Phe Asn Gln Leu Lys Ala
        195                 200                 205

CTC AAC GCC GAG CGC AGC ATC CTC TCC CAG AGC TTC GAC TAC AAC TAC 672
Leu Asn Ala Glu Arg Ser Ile Leu Ser Gln Ser Phe Asp Tyr Asn Tyr
    210                 215                 220
GGC GAC TTC ATC CCC GTC CTC CGC CCC TTC CTC CGC CGC TAC CTC AAC 720
Gly Asp Phe Ile Pro Val Leu Arg Pro Phe Leu Arg Arg Tyr Leu Asn
225                 230                 235                 240

CGC TGC ACC AAC CTC AAG ACC AAG CGG ATG AAG GTG TTC GAG GAC CAC 768
Arg Cys Thr Asn Leu Lys Thr Lys Arg Met Lys Val Phe Glu Asp His
                245                 250                 255

TTC GTC CAG CAG CGC AAG GAG GCG TTG GAG AAG ACG GGT GAG ATC AGG 816
Phe Val Gln Gln Arg Lys Glu Ala Leu Glu Lys Thr Gly Glu Ile Arg
            260                 265                 270

TGC GCC ATG GAC CAC ATC CTG GAA GCC GAA AGG AAG GGC GAG ATC AAC 864
Cys Ala Met Asp His Ile Leu Glu Ala Glu Arg Lys Gly Glu Ile Asn
        275                 280                 285

CAC GAC AAC GTC CTC TAC ATC GTC GAG AAC ATC AAC GTC GCA GCC ATC 912
His Asp Asn Val Leu Tyr Ile Val Glu Asn Ile Asn Val Ala Ala Ile
    290                 295                 300

GAG ACG ACG CTG TGG TCG ATC GAG TGG GGC CTC GCG GAG CTG GTG AAC 960
Glu Thr Thr Leu Trp Ser Ile Glu Trp Gly Leu Ala Glu Leu Val Asn
305                 310                 315                 320

CAC CCG GAG ATC CAG CAG AAG CTG CGC GAG GAG ATC GTC GCC GTT CTG 1008
His Pro Glu Ile Gln Gln Lys Leu Arg Glu Glu Ile Val Ala Val Leu
                325                 330                 335

GGC GCC GGC GTG GCG GTG ACG GAG CCG GAC CTG GAG CGC CTC CCC TAC 1056
Gly Ala Gly Val Ala Val Thr Glu Pro Asp Leu Glu Arg Leu Pro Tyr
            340                 345                 350

CTG CAG TCC GTG GTG AAG GAG ACG CTC CGC CTC CGC ATG GCA ATC CCG 1104
Leu Gln Ser Val Val Lys Glu Thr Leu Arg Leu Arg Met Ala Ile Pro
        355                 360                 365

CTC CTG GTG CCG CAC ATG AAC CTC AGC GAC GCC AAG CTC GCC GGC TAC 1152
Leu Leu Val Pro His Met Asn Leu Ser Asp Ala Lys Leu Ala Gly Tyr
    370                 375                 380

GAC ATC CCC GCC GAG TCC AAG ATC CTC GTC AAC GCC TGG TTC CTC GCC 1200
Asp Ile Pro Ala Glu Ser Lys Ile Leu Val Asn Ala Trp Phe Leu Ala
385                 390                 395                 400

AAC GAC CCC AAG CGG TGG GTG CGC GCC GAT GAG TTC AGG CCG GAG AGG 1248
Asn Asp Pro Lys Arg Trp Val Arg Ala Asp Glu Phe Arg Pro Glu Arg
                405                 410                 415

TTC CTC GAG GAG GAG AAG GCC GTC GAG GCC CAC GGC AAC GAT TTC CGG 1296
Phe Leu Glu Glu Glu Lys Ala Val Glu Ala His Gly Asn Asp Phe Arg
            420                 425                 430

TTC GTG CCC TTC GGC GTC GGC CGC CGG AGC TGC CCC GGG ATC ATC CTC 1344
Phe Val Pro Phe Gly Val Gly Arg Arg Ser Cys Pro Gly Ile Ile Leu
        435                 440                 445

GCG CTG CCC ATC ATC GGC ATC ACG CTC GGA CGC CTG GTG CAG AAC TTC     1392
Ala Leu Pro Ile Ile Gly Ile Thr Leu Gly Arg Leu Val Gln Asn Phe
    450                 455                 460

CAG CTG CTG CCG CCG CCG GGG CAG GAC AAG ATC GAC ACC ACC GAG AAG     1440
Gln Leu Leu Pro Pro Pro Gly Gln Asp Lys Ile Asp Thr Thr Glu Lys
465                 470                 475                 480

CCC GGG CAG TTT ACC AAC CAG ATC CTC AAG CAC GCC ACC ATT GTC TGC     1488
Pro Gly Gln Phe Thr Asn Gln Ile Leu Lys His Ala Thr Ile Val Cys
                485                 490                 495

AAG CCA CTC GAG GCT TAA                                             1506
Lys Pro Leu Glu Ala
            500

(2) INFORMATIONS POUR LA SEQ ID NO: 10:

    (i) CARACTERISTIQUES DE LA SEQUENCE:
        (A) LONGUEUR: 2181 paires de bases
        (B) TYPE: nucléotide
        (C) NOMBRE DE BRINS: simple
        (D) CONFIGURATION: linéaire

    (ii) TYPE DE MOLECULE: ADNc

    (ix) CARACTERISTIQUE:
        (A) NOM/CLE: CDS
        (B) EMPLACEMENT:112..1734

    (xi) DESCRIPTION DE LA SEQUENCE: SEQ ID NO: 10:

CGATCCACCC CTTGGATCCA CTCTACCCAG CTCGCTAGCC AGCGGGGTAC ATACACGCAC      60

GCACGTACGC GCGTACGTAC ACTCGCAGAG CTTGCTTCAG GGAGGCCGGC A ATG GAG     117
                                                         Met Glu
                                                           1

GTG GGG ACG TGG GCG GTG GTG GTG TCG GCG GTG GCC GCG TAC ATG GCG     165
Val Gly Thr Trp Ala Val Val Val Ser Ala Val Ala Ala Tyr Met Ala
        5                  10                  15

TGG TTC TGG CGG ATG TCC CGC GGG CTG CGC GGG CCG CGG GTT TGG CCC     213
Trp Phe Trp Arg Met Ser Arg Gly Leu Arg Gly Pro Arg Val Trp Pro
    20                  25                  30

GTG CTC GGC AGC CTG CCG GGC CTG GTG CAG CAC GCC GAG GAC ATG CAC     261
Val Leu Gly Ser Leu Pro Gly Leu Val Gln His Ala Glu Asp Met His
 35                  40                  45                  50

GAG TGG ATC GCC GGC AAC CTG CGC CGC GCG GGC GGC ACG TAC CAG ACC     309
Glu Trp Ile Ala Gly Asn Leu Arg Arg Ala Gly Gly Thr Tyr Gln Thr
                 55                  60                  65

TGC ATC TTC GCC GTG CCC GGG GTG GCG CGC CGC GGC GGC CTG GTC ACC     357
Cys Ile Phe Ala Val Pro Gly Val Ala Arg Arg Gly Gly Leu Val Thr
             70                  75                  80

GTC ACC TGC GAC CCG CGC AAC CTG GAG CAC GTC CTG AAG GCG CGC TTC     405
Val Thr Cys Asp Pro Arg Asn Leu Glu His Val Leu Lys Ala Arg Phe
         85                  90                  95

GAC AAC TAC CCC AAG GGC CCC TTC TGG CAC GGC GTC TTC CGG GAC CTG     453
Asp Asn Tyr Pro Lys Gly Pro Phe Trp His Gly Val Phe Arg Asp Leu
    100                 105                 110

CTC GGC GAC GGC ATC TTC AAT TCC GAC GGC GAC ACC TGG CTC GCG CAG 501
Leu Gly Asp Gly Ile Phe Asn Ser Asp Gly Asp Thr Trp Leu Ala Gln
115                 120                 125                 130

CGC AAG ACG GCC GCG CTC GAG TTC ACC ACC CGC ACG CTC CGG ACG GCC 549
Arg Lys Thr Ala Ala Leu Glu Phe Thr Thr Arg Thr Leu Arg Thr Ala
                135                 140                 145

ATG TCC CGC TGG GTC TCG CGC TCC ATC CAC GGC CGC CTC CTG CCC ATC 597
Met Ser Arg Trp Val Ser Arg Ser Ile His Gly Arg Leu Leu Pro Ile
            150                 155                 160

CTG GCC GAC GCG GCC AAG GGC AAG GCG CAG GTG GAT CTC CAG GAC CTC 645
Leu Ala Asp Ala Ala Lys Gly Lys Ala Gln Val Asp Leu Gln Asp Leu
        165                 170                 175

CTC CTC CGC CTC ACC TTC GAC AAC ATC TGC GGC CTG GCC TTC GGC AAG 693
Leu Leu Arg Leu Thr Phe Asp Asn Ile Cys Gly Leu Ala Phe Gly Lys
    180                 185                 190

GAC CCG GAG ACG CTC GCC CAG GGC CTG CCG GAG AAC GAG TTC GCC TCC 741
Asp Pro Glu Thr Leu Ala Gln Gly Leu Pro Glu Asn Glu Phe Ala Ser
195                 200                 205                 210

GCG TTC GAC CGC GCC ACC GAG GCC ACG CTC AAC CGC TTC ATC TTC CCG 789
Ala Phe Asp Arg Ala Thr Glu Ala Thr Leu Asn Arg Phe Ile Phe Pro
                215                 220                 225

GAG TTC CTG TGG CGC TGC AAA AAG TGG CTG GGC CTC GGC ATG GAG ACC 837
Glu Phe Leu Trp Arg Cys Lys Lys Trp Leu Gly Leu Gly Met Glu Thr
            230                 235                 240

ACG CTG ACC AGC AGC ATG GCC CAC GTC GAC CAG TAC CTC GCC GCC GTC 885
Thr Leu Thr Ser Ser Met Ala His Val Asp Gln Tyr Leu Ala Ala Val
        245                 250                 255

ATC AAG AAG CGC AAG CTC GAG CTC GCC GCC GGC AAC GGC AAA TGC GAC 933
Ile Lys Lys Arg Lys Leu Glu Leu Ala Ala Gly Asn Gly Lys Cys Asp
    260                 265                 270

ACG GCG GCG ACG CAC GAC GAC CTG CTC TCC CGG TTC ATG CGG AAG GGT 981
Thr Ala Ala Thr His Asp Asp Leu Leu Ser Arg Phe Met Arg Lys Gly
275                 280                 285                 290

TCC TAC TCG GAC GAG TCG CTC CAG CAC GTG GCG CTC AAC TTC ATC CTC 1029
Ser Tyr Ser Asp Glu Ser Leu Gln His Val Ala Leu Asn Phe Ile Leu
                295                 300                 305

GCC GGC CGC GAC ACC TCC TCC GTG GCG CTC TCC TGG TTC TTC TGG CTC 1077
Ala Gly Arg Asp Thr Ser Ser Val Ala Leu Ser Trp Phe Phe Trp Leu
            310                 315                 320

GTG TCC ACC CAC CCT GCG GTG GAG CGC AAG ATC GTG CGC GAG CTC TGC 1125
Val Ser Thr His Pro Ala Val Glu Arg Lys Ile Val Arg Glu Leu Cys
        325                 330                 335

TCC GTT CTC GCC GCG TCA CGG GGC GCC CAT GAC CCG GCA TTG TGG CTG 1173
Ser Val Leu Ala Ala Ser Arg Gly Ala His Asp Pro Ala Leu Trp Leu
    340                 345                 350

GCG GAG CCC TTC ACC TTC GAG GAG CTC GAC CGC CTG GTC TAC CTC AAG 1221
Ala Glu Pro Phe Thr Phe Glu Glu Leu Asp Arg Leu Val Tyr Leu Lys
355                 360                 365                 370

GCG GCG CTG TCG GAG ACC CTC CGC CTC TAC CCC TCC GTC CCC GAG GAC 1269
Ala Ala Leu Ser Glu Thr Leu Arg Leu Tyr Pro Ser Val Pro Glu Asp
                375                 380                 385

TCC AAG CAC GTC GTC GCG GAC GAC TAC CTC CCC GAC GGC ACC TTC GTG 1317
Ser Lys His Val Val Ala Asp Asp Tyr Leu Pro Asp Gly Thr Phe Val
            390                 395                 400

CCG GCC GGG TCG TCG GTC ACC TAC TCC ATA TAC TCG GCG GGG CGC ATG 1365
Pro Ala Gly Ser Ser Val Thr Tyr Ser Ile Tyr Ser Ala Gly Arg Met
        405                 410                 415

AAG GGG GTG TGG GGG GAG GAC TGC CTC GAG TTC CGG CCG GAG CGA TGG 1413
Lys Gly Val Trp Gly Glu Asp Cys Leu Glu Phe Arg Pro Glu Arg Trp
    420                 425                 430

CTG TCG GCC GAC GGC ACC AAG TTC GAG CAG CAC GAC TCG TAC AAG TTC 1461
Leu Ser Ala Asp Gly Thr Lys Phe Glu Gln His Asp Ser Tyr Lys Phe
435                 440                 445                 450

GTG GCG TTC AAC GCC GGG CCG AGG GTG TGC CTG GGC AAG GAC CTA GCC 1509
Val Ala Phe Asn Ala Gly Pro Arg Val Cys Leu Gly Lys Asp Leu Ala
                455                 460                 465

TAC CTG CAG ATG AAG AAC ATC GCC GGG AGC GTG CTG CTC CGG CAC CGC 1557
Tyr Leu Gln Met Lys Asn Ile Ala Gly Ser Val Leu Leu Arg His Arg
            470                 475                 480

CTG ACC GTG GCG CCG GGC CAC CGC GTG GAG CAG AAG ATG TCG CTC ACG 1605
Leu Thr Val Ala Pro Gly His Arg Val Glu Gln Lys Met Ser Leu Thr
        485                 490                 495

CTC TTC ATG AAG GGC GGG CTA CGG ATG GAG GTA CGT CCG CGC GAC CTC 1653
Leu Phe Met Lys Gly Gly Leu Arg Met Glu Val Arg Pro Arg Asp Leu
    500                 505                 510

GCC CCC GTC CTC GAC GAG CCC TGC GGC CTG GAC GCC GGC GCC GCC ACC 1701
Ala Pro Val Leu Asp Glu Pro Cys Gly Leu Asp Ala Gly Ala Ala Thr
515                 520                 525                 530

GCC GCC GCA GCA AGT GCC ACA GCG CCG TGC GCG TAGAAGACCT GGCACCGGCA 1754
Ala Ala Ala Ala Ser Ala Thr Ala Pro Cys Ala
                535                 540

CGCGCCATGC ATGATTCGTG CGTGCTAGCT GTTGAAGGGA CGCCGGACAT TGAATGTGTA 1814

GATAGGGCAG CAGTGCAAGA CCGTAAGTAA AATTGATGAT GGGTTTGGTG ACAACATTGA 1874

AGCCACTCCT TTCCAGAATT TACGACCCGG ATAGGAGAAA CAGGGAAACT TTGCAGATCA 1934

CAACACAAGA TCTAGCCAGC CGGGGATCTG ATCTGATTTG CGTCTGCTCG GAGCACGGGT 1994

GCATGGGAGA CCAAGGAGGA AAACAAAAAA TAACAGAAAC AGAGTGAGCA ATATTTGTGA 2054

TTGTAGCCAC GGGAAAGAGA GAGGAGTAAT TAGTAATTCA GATTTGTTTG CAGTAGCTCG 2114

GTGTTGGTGA CCAGATCATA GCCAACTAGG CTATTCTATT CTATTCTATT TTTGAAGATG 2174

ATTTTTC 2181

(2) INFORMATIONS POUR LA SEQ ID NO: 11:

(i) CARACTERISTIQUES DE LA SEQUENCE:
(A) LONGUEUR: 150 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "Amorce"

(xi) DESCRIPTION DE LA SEQUENCE: SEQ ID NO: 11:

ATATATGGAT CCATGGAGGT GGGGACGTGG GCGGTGGTG 39

(2) INFORMATIONS POUR LA SEQ ID NO: 12:

(i) CARACTERISTIQUES DE LA SEQUENCE:
(A) LONGUEUR: 150 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "Amorce"

(xi) DESCRIPTION DE LA SEQUENCE: SEQ ID NO: 12:

ATATATGGAT CCATGGAAGT TGGTACTTGG GCTGTTGTTG TTTCTGCTGT TGCTGCTTAT 60

ATGGCTTGGT TTTGGAGAAT GTCTAGAGGT TTGAGAGGTC CAAGAGTTTG GCCAGTTTTG 120

GGTTCTTTGC CAGGCCTGGT GCAGCACGCC 150

(2) INFORMATIONS POUR LA SEQ ID NO: 13:

(i) CARACTERISTIQUES DE LA SEQUENCE:
(A) LONGUEUR: 42 paires de bases
(B) TYPE: nucléotide
(C) NOMBRE DE BRINS: simple
(D) CONFIGURATION: linéaire

(ii) TYPE DE MOLECULE: Autre acide nucléique
(A) DESCRIPTION: /desc = "Reverse"

(xi) DESCRIPTION DE LA SEQUENCE: SEQ ID NO: 13:

TATATAGAAT TCCTTCTACG CGCACGGCGC TGTGGCACTT GC 42

(2) INFORMATIONS POUR LA SEQ ID NO: 14:

(i) CARACTERISTIQUES DE LA SEQUENCE:
(A) LONGUEUR: 1626 paires de bases
(B) TYPE: nucléotide
(C) NOMBRE DE BRINS: simple
(D) CONFIGURATION: linéaire

(ii) TYPE DE MOLECULE: ADNc

(ix) CARACTERISTIQUE:
(A) NOM/CLE: CDS
(B) EMPLACEMENT:1..1623

(xi) DESCRIPTION DE LA SEQUENCE: SEQ ID NO: 14:

ATG GAA GTT GGT ACT TGG GCT GTT GTT GTT TCT GCT GTT GCT GCT TAT 48
Met Glu Val Gly Thr Trp Ala Val Val Val Ser Ala Val Ala Ala Tyr
1               5                   10                  15

ATG GCT TGG TTT TGG AGA ATG TCT AGA GGT TTG AGA GGT CCA AGA GTT 96
Met Ala Trp Phe Trp Arg Met Ser Arg Gly Leu Arg Gly Pro Arg Val
            20                  25                  30

TGG CCA GTT TTG GGT TCT TTG CCA GGC CTG GTG CAG CAC GCC GAG GAC 144
Trp Pro Val Leu Gly Ser Leu Pro Gly Leu Val Gln His Ala Glu Asp
        35                  40                  45

ATG CAC GAG TGG ATC GCC GGC AAC CTG CGC CGC GCG GGC GGC ACG TAC 192
Met His Glu Trp Ile Ala Gly Asn Leu Arg Arg Ala Gly Gly Thr Tyr
    50                  55                  60

CAG ACC TGC ATC TTC GCC GTG CCC GGG GTG GCG CGC CGC GGC GGC CTG 240
Gln Thr Cys Ile Phe Ala Val Pro Gly Val Ala Arg Arg Gly Gly Leu
65                  70                  75                  80

GTC ACC GTC ACC TGC GAC CCG CGC AAC CTG GAG CAC GTC CTG AAG GCG 288
Val Thr Val Thr Cys Asp Pro Arg Asn Leu Glu His Val Leu Lys Ala
                85                  90                  95

CGC TTC GAC AAC TAC CCC AAG GGC CCC TTC TGG CAC GGC GTC TTC CGG 336
Arg Phe Asp Asn Tyr Pro Lys Gly Pro Phe Trp His Gly Val Phe Arg
            100                 105                 110

GAC CTG CTC GGC GAC GGC ATC TTC AAT TCC GAC GGC GAC ACC TGG CTC 384
Asp Leu Leu Gly Asp Gly Ile Phe Asn Ser Asp Gly Asp Thr Trp Leu
        115                 120                 125

GCG CAG CGC AAG ACG GCC GCG CTC GAG TTC ACC ACC CGC ACG CTC CGG 432
Ala Gln Arg Lys Thr Ala Ala Leu Glu Phe Thr Thr Arg Thr Leu Arg
    130                 135                 140

ACG GCC ATG TCC CGC TGG GTC TCG CGC TCC ATC CAC GGC CGC CTC CTG 480
Thr Ala Met Ser Arg Trp Val Ser Arg Ser Ile His Gly Arg Leu Leu
145                 150                 155                 160

CCC ATC CTG GCC GAC GCG GCC AAG GGC AAG GCG CAG GTG GAT CTC CAG 528
Pro Ile Leu Ala Asp Ala Ala Lys Gly Lys Ala Gln Val Asp Leu Gln
                165                 170                 175

GAC CTC CTC CTC CGC CTC ACC TTC GAC AAC ATC TGC GGC CTG GCC TTC 576
Asp Leu Leu Leu Arg Leu Thr Phe Asp Asn Ile Cys Gly Leu Ala Phe
            180                 185                 190

GGC AAG GAC CCG GAG ACG CTC GCC CAG GGC CTG CCG GAG AAC GAG TTC 624
Gly Lys Asp Pro Glu Thr Leu Ala Gln Gly Leu Pro Glu Asn Glu Phe
        195                 200                 205

GCC TCC GCG TTC GAC CGC GCC ACC GAG GCC ACG CTC AAC CGC TTC ATC 672
Ala Ser Ala Phe Asp Arg Ala Thr Glu Ala Thr Leu Asn Arg Phe Ile
    210                 215                 220

TTC CCG GAG TTC CTG TGG CGC TGC AAA AAG TGG CTG GGC CTC GGC ATG 720
Phe Pro Glu Phe Leu Trp Arg Cys Lys Lys Trp Leu Gly Leu Gly Met
225                 230                 235                 240

GAG ACC ACG CTG ACC AGC AGC ATG GCC CAC GTC GAC CAG TAC CTC GCC 768
Glu Thr Thr Leu Thr Ser Ser Met Ala His Val Asp Gln Tyr Leu Ala
                245                 250                 255

GCC GTC ATC AAG AAG CGC AAG CTC GAG CTC GCC GCC GGC AAC GGC AAA 816
Ala Val Ile Lys Lys Arg Lys Leu Glu Leu Ala Ala Gly Asn Gly Lys
            260                 265                 270

TGC GAC ACG GCG GCG ACG CAC GAC GAC CTG CTC TCC CGG TTC ATG CGG 864
Cys Asp Thr Ala Ala Thr His Asp Asp Leu Leu Ser Arg Phe Met Arg
        275                 280                 285

AAG GGT TCC TAC TCG GAC GAG TCG CTC CAG CAC GTG GCG CTC AAC TTC 912
Lys Gly Ser Tyr Ser Asp Glu Ser Leu Gln His Val Ala Leu Asn Phe
    290                 295                 300

ATC CTC GCC GGC CGC GAC ACC TCC TCC GTG GCG CTC TCC TGG TTC TTC 960
Ile Leu Ala Gly Arg Asp Thr Ser Ser Val Ala Leu Ser Trp Phe Phe
305                 310                 315                 320

TGG CTC GTG TCC ACC CAC CCT GCG GTG GAG CGC AAG ATC GTG CGC GAG 1008
Trp Leu Val Ser Thr His Pro Ala Val Glu Arg Lys Ile Val Arg Glu
                325                 330                 335

CTC TGC TCC GTT CTC GCC GCG TCA CGG GGC GCC CAT GAC CCG GCA TTG 1056
Leu Cys Ser Val Leu Ala Ala Ser Arg Gly Ala His Asp Pro Ala Leu
            340                 345                 350

TGG CTG GCG GAG CCC TTC ACC TTC GAG GAG CTC GAC CGC CTG GTC TAC 1104
Trp Leu Ala Glu Pro Phe Thr Phe Glu Glu Leu Asp Arg Leu Val Tyr
        355                 360                 365

CTC AAG GCG GCG CTG TCG GAG ACC CTC CGC CTC TAC CCC TCC GTC CCC 1152
Leu Lys Ala Ala Leu Ser Glu Thr Leu Arg Leu Tyr Pro Ser Val Pro
    370                 375                 380

GAG GAC TCC AAG CAC GTC GTC GCG GAC GAC TAC CTC CCC GAC GGC ACC 1200
Glu Asp Ser Lys His Val Val Ala Asp Asp Tyr Leu Pro Asp Gly Thr
385                 390                 395                 400

TTC GTG CCG GCC GGG TCG TCG GTC ACC TAC TCC ATA TAC TCG GCG GGG 1248
Phe Val Pro Ala Gly Ser Ser Val Thr Tyr Ser Ile Tyr Ser Ala Gly
                405                 410                 415

CGC ATG AAG GGG GTG TGG GGG GAG GAC TGC CTC GAG TTC CGG CCG GAG 1296
Arg Met Lys Gly Val Trp Gly Glu Asp Cys Leu Glu Phe Arg Pro Glu
            420                 425                 430

CGA TGG CTG TCG GCC GAC GGC ACC AAG TTC GAG CAG CAC GAC TCG TAC 1344
Arg Trp Leu Ser Ala Asp Gly Thr Lys Phe Glu Gln His Asp Ser Tyr
        435                 440                 445

AAG TTC GTG GCG TTC AAC GCC GGG CCG AGG GTG TGC CTG GGC AAG GAC 1392
Lys Phe Val Ala Phe Asn Ala Gly Pro Arg Val Cys Leu Gly Lys Asp
    450                 455                 460

CTA GCC TAC CTG CAG ATG AAG AAC ATC GCC GGG AGC GTG CTG CTC CGG 1440
Leu Ala Tyr Leu Gln Met Lys Asn Ile Ala Gly Ser Val Leu Leu Arg
465                 470                 475                 480

CAC CGC CTG ACC GTG GCG CCG GGC CAC CGC GTG GAG CAG AAG ATG TCG 1488
His Arg Leu Thr Val Ala Pro Gly His Arg Val Glu Gln Lys Met Ser
                485                 490                 495

CTC ACG CTC TTC ATG AAG GGC GGG CTA CGG ATG GAG GTA CGT CCG CGC 1536
Leu Thr Leu Phe Met Lys Gly Gly Leu Arg Met Glu Val Arg Pro Arg
            500                 505                 510

GAC CTC GCC CCC GTC CTC GAC GAG CCC TGC GGC CTG GAC GCC GGC GCC 1584
Asp Leu Ala Pro Val Leu Asp Glu Pro Cys Gly Leu Asp Ala Gly Ala
        515                 520                 525

GCC ACC GCC GCC GCA GCA AGT GCC ACA GCG CCG TGC GCG TAG 1626
Ala Thr Ala Ala Ala Ala Ser Ala Thr Ala Pro Cys Ala
    530                 535                 540